Europäisches Patentamt

European Patent Office

Office européen des brevets

(19)

(11) Numéro de publication: **0 101 363**
**A1**

(12) ## DEMANDE DE BREVET EUROPEEN

(21) Numéro de dépôt: **83401579.4**

(22) Date de dépôt: **29.07.83**

(51) Int. Cl.³: **A 61 K 39/39**

(30) Priorité: **30.07.82 FR 8213409**

(43) Date de publication de la demande:
**22.02.84 Bulletin 84/8**

(84) Etats contractants désignés:
**AT BE CH DE GB IT LI LU NL SE**

(71) Demandeur: **ANVAR Agence Nationale de Valorisation de la Recherche**
**43, rue de Caumartin**
**F-75436 Paris Cedex 09(FR)**

(72) Inventeur: **Bahr, Georges**
**84, Boulevard Pasteur**
**F-75015 Paris(FR)**

(72) Inventeur: **Chedid, Louis**
**16-18, rue Gaston de Caillavet**
**F-75015 Paris(FR)**

(74) Mandataire: **Gutmann, Ernest et al,**
**Cabinet Plasseraud 84, rue d'Amsterdam**
**F-75009 Paris(FR)**

(54) **Procédé de séparation de facteurs ayant des propriétés d'activation des lymphocytes à partir de milieu d'incubation de populations cellulaires, notamment de lignée granulocyto-macrophagique, par une réaction antigène-anticorps mettant en jeu des anticorps anti-MDP.**

(57) L'invention concerne un procédé de séparation ou de purification d'un facteur LAF, ayant des propriétés d'activation des lymphocytes et, le cas échéant, des propriétés pyrogènes-endogènes, à partir d'un milieu biologique liquide le contenant à l'état dissous, ou d'enrichissement de ce milieu en ce facteur LAF. Ce procédé est caractérisé par la mise en contact de ce milieu avec des anticorps anti-MDP, et la récupération soit du facteur LAF lui-même, à partir du complexe facteur LAF-anticorps anti-MDP, soit du milieu alors substantiellement dépourvu de ce facteur.

EP 0 101 363 A1

Croydon Printing Company Ltd.

1

Procédé de séparation de facteurs ayant des propriétés d'activation des lymphocytes à partir de milieu d'incubation de populations cellulaires, notamment de lignée granulocyto-macrophagique, par une réaction antigène-anticorps mettant en jeu des anticorps anti-MDP.

———

L'invention est relative à un procédé de séparation de facteurs ayant des propriétés d'activation de lymphocytes, parmi lesquels également des facteurs présentant des propriétés pyrogènes-endogènes, à partir d'un milieu d'incubation de populations cellulaires, de lignée granulocyto-macrophagique, par une réaction antigène-anticorps mettant en jeu des anticorps anti-MDP.

On sait que l'administration d'endotoxines et de nombreux autres produits bactériens est susceptible d'induire la production par des leucocytes de facteurs ayant des propriétés d'activation ou de stimulation des lymphocytes plus particulièrement des lymphocytes T (activité de type "Lymphocyte Activating Factor" ou "LAF"). Parmi les facteurs ayant ce type d'activité figurent également des pyrogènes endogènes PE obtenus à partir de leucocytes. Ces facteurs sont tenus pour responsables en grande partie de l'élévation de température induite *in vivo* par l'administration desdites endotoxines et autres produits bactériens.

Le N-acétylmuramyl-L-alanyl-D-isoglutamine, ou MDP (pour muramyl dipeptide), a été identifié comme étant la structure minimale des peptidoglycanes bactériens capable de remplacer les mycobactéries entières dans l'adjuvant complet de Freund (ACF). Par ailleurs, cet adjuvant, qui peut être obtenu par synthèse, s'est révélé capable de produire une hyperthermie chez le lapin, soit en libérant des pyrogènes leucocytaires circulants, soit par action directe sur les centres de la thermorégulation. Il est à noter que la dose minimale pyrogène de MDP est de 30 microgrammes/kg, lorsque le produit est injecté par voie intraveineuse, alors que, après administration par voie intracérébroventriculaire, la dose de 0,1 nanogramme, soit environ 20 picomoles est active. Cette dose est voisine de celle à laquelle le pyrogène endogène est théoriquement actif.

Des facteurs ayant une activité du type LAF, et notamment de tels facteurs qui possèdent des propriétés pyrogènes-endogènes, peuvent également être induites *in vitro*. En particulier, les susdites populations cellulaires, notamment celles appartenant aux séries monocytaires et lymphocytaires, sont susceptibles de libérer des facteurs ou cytokines, par exemple lymphokines ou monokines, lorsqu'elles sont incubées dans un milieu de culture contenant un agent immunostimulant. Ces facteurs, qui sont libérés dans le milieu de culture, peuvent être isolés à partir de celui-ci. Parmi ces facteurs peuvent également figurer les pyrogènes-endogènes, notamment ceux qui sont également connus sous la désignation Interleukines 1 (IL-1).

De nombreux agents immuno-stimulants peuvent être mis en eouvre, pour induire la production *in vitro* desdites interleukines 1. On mentionnera par exemple des lectines, des produits naturels notamment d'origine virale, bactérienne, fongique ou parasitaire, plus particulièrement des produits provenant de parois bactériennes tels que les produits du type lipopolysaccharide bactérien (LPS). On peut aussi mettre en oeuvre des micro-organismes entiers. Des Il-1 peuvent être induites dans des cultures de cellules mononucléées humaines du sang périphérique, stimulées pendant une heure à l'aide de staphylocoques tués par la chaleur. Après lavage, les cellules adhérentes sont incubées 24 heures, puis leur surnageant de culture contenant les pyrogènes leucocytaires, ci-après désignés par l'abréviation "PL", est centrifugé à 25.000 G et conservé à 4°C.

Il a également été montré que le MDP et de nombreux homologues du MDP pouvaient être utilisés pour induire la production *in vitro* des IL-1 susdites. Les fractions LAF qui peuvent ainsi être isolées des milieux de culture stimulés présentent également une activité pyrogène. Cependant lorsque l'on a recours à une classe préférée d'homologues du MDP, plus particulièrement aux dérivés α-esters de la N-acétyl-muramyl-L-alanyl-D-glutamine, tels que décrits notamment dans la demande de brevet européen n° 79400357 et de préférence à l'α-nbutyl-ester de la N-acétyl-muramyl-L-alanyl-D-glutamine, il est possible d'induire dans lesdites cultures cellulaires la production de monokines présentant les activités immuno-stimulantes caractéristiques du LAF, ces monokines étant cependant dépourvues de pyrogénicité-endogène. On pourra se rapporter avec avantage, pour la description des conditions dans lesquelles cette induction peut être réalisée, à la demande de brevet français n° 82 00241.

Des procédés de séparation, voire de purification mettant en jeu des réactions antigène-anticorps ont déjà été décrits. Ainsi on a déjà décrit un procédé d'obtention d'une fraction d'immunoglobulines anti-pyrogène, par immunisation du lapin avec un surnageant d'une culture de leucocytes humains antérieurement stimulés et doués d'activité pyrogène (DINARELLO, C.A., RENFER, L. et WOLFF, S.M., J. Clin. Invest.,vol.60,1977,p.465-472).

De même l'utilisation de ces anticorps pour constituer des colonnes de chromatographie d'affinité, par fixation sur le matériau immunoabsorbant connu sous la désignation SEPHAROSE 4B, activé au préalable par le bromure de cyanogène a également été décrite par ANFINSEN et Coll. dans "Proc. Natl. Acad. Sci. USA",vol.71,p.3139-3142.

Ce sont cesanticorps qui seront dans ce qui suit désignés par l'expression "anti-PL humain".

Ces anticorps fixés sur SEPHAROSE 4B sont à l'origine d'un procédé connu pour purifier le PL, ou plus généralement lespyrogènes-endogènes (PE) issus de toute autre source. Ce procédé comprend notamment les étapes consistant à mettre en contact les surnageants préalablement concentrés, par exemple dans les conditions déjà indiquées, avec une colonne immunoabsorbante de SEPHAROSE 4B activé par le bromure de cyanogène et portant les anticorps de lapin anti-PL-humains, à éluer le PL, à soumettre l'éluat à une chromatographie sur une colonne du tamis moléculaire,commercialisé sous la désignation SEPHADEX G50, et à recueillir les fractions ayant un poids moléculaire de l'ordre de 15.000, donnant une bande unique sur gel de polyacrylamide contenant 7,5 % de sodium-dodécyl sulfate (SDS), et douées d'activité pyrogène et d'activité LAF.

Les fractions PL ainsi obtenues, certes concentrées en pyrogène-leucocytaire, contiennent cependant toujours encore de quantités substantielles d'autres facteurs, notamment lymphokines, monokines et interféron α, dont la séparation par les procédés classiques n'est guère permise.

L'invention a pour but de remédier à cette difficulté, plus particulièrement de fournir un procédé plus sélectif de séparation à partir des susdits milieux ou, selon le cas, de purification des facteurs présentant une activité du type LAF, ci-après désignés sous l'expression "facteurs LAF" des PL ou, plus généralement, des fractions pyrogènes-endogènes, ci-après désignées sous l'abréviation PE, également douées des propriétés LAF.

L'invention a encore pour but, à l'inverse, de fournir un procédé d'élimination des PE, à partir de tout milieu susceptible d'en contenir, en vue de l'obtention d'un milieu substantiellement dépourvu de pyrogènes-endogènes et à partir duquel pourront ensuite être obtenus d'autres principes actifs essentiellement dépourvus de PE.

L'invention découle de la découverte que des facteurs ayant des propriétés du type LAF, et parmi eux les facteurs pyrogènes-endogènes, pouvaient être reconnus de façon sélective par des anticorps anti-MDP spécifiques.

Le procédé selon l'invention de séparation ou de purification des facteurs ayant une activité de type LAF, et notamment de PE, à partir d'un milieu biologique liquide les contenant à l'état dissous, est caractérisé par la mise en contact de ce milieu avec des anticorps anti-MDP, et la récupération à partir du complexe facteur PE-anticorps anti-MDP, du facteur PE lui-même.

Les anti-corps anti-MDP susceptibles d'être mis en oeuvre dans le procédé de purification selon l'invention peuvent être obtenus de toute façon en soi connue, notamment par immunisation de l'animal, par exemple le lapin, avec un MDP ou dérivé de MDP préalablement fixé sur une molécule porteuse, telle que la sérumalbumine bovine, des polylysines ou autre molécule ayant un poids moléculaire suffisamment élevé pour conférer au conjugué obtenu l'immunogénicité requise. En ce qui concerne les conditions générales de la fabrication des anticorps anti-MDP, on peut se référer à l'article de REICHERT C. M. et coll. publié dans Molec. Immun. 17, 357-363 (1980). On peut aussi se reporter à l'article de BAHR G. M. et coll. publié dans Molec. Immun. 19, n° 5, 737-745 (1982).

Les anticorps contenus dans les antisérums obtenus peuvent être purifiés, par exemple par chromatographie affine sur une colonne de résine portant des molécules de MDP ou dérivées de MDP fixées sur cette résine. Avantageusement, on aura recours à une colonne du matériau support insoluble commercialisé sous la désignation SEPHAROSE 4B sur laquelle ont été fixés des groupes MDP-lysine (N-acétyl-muramyl-L-alanyl-D-isoglutaminyl-L-lysine), après activation préalable du matériau support au bromure de cyanogène.

Les anticorps anti-MDP (anticorps anti-MDP polyclonaux) fixés sur un tel matériau peuvent ensuite être élués par exemple par acidification du milieu, notamment à un pH inférieur à 4, ou par accroissement de la concentration ionique du milieu, grâce à des sels ioniques fortement solubles dans l'eau, tels que le thiocyanate de sodium.

Selon une caractéristique préférée importante de l'invention, on a cependant recours à des anticorps anti-MDP "sélectifs" en ce qu'ils reconnaissent de façon spécifique la structure N-acétyl-muramyl-L-alanyl-D-isoglutamine dans son ensemble, à l'exclusion notamment soit de la structure de l'acide N-acétyl-muramique isolé, soit de celle de la L-alanyl-D-isoglutamine isolée.

Ces derniers anticorps anti-MDP sélectifs, plus particulièrement des anticorps monoclonaux, permettront un degré de purification nettement plus poussé que les anticorps polyclonaux, puisque ces derniers sont susceptibles de fixer d'autres constituants des milieux biologiques concernés, par exemple de nombreuses autres structures glycopeptides également présentes dans ces milieux. Néanmoins les anticorps anti-MDP polyclonaux peuvent être utilisés avec avantage pour produire un premier enrichissement en facteur PE, des purifications plus poussées étant ensuite, si besoin, répétées en mettant en oeuvre des anticorps "sélectifs" monoclonaux. Une technique d'obtention d'hybridomes secrétant de tels anticorps monoclonaux et à la récupérer de ceux-ci à partir des milieux de cultures de ces hybridomes, ainsi que les conditions dans lesquelles ils peuvent être mis en oeuvre, seront indiquées plus loin, exclusivement à titre d'exemples.

Le procédé selon l'invention présente donc les caractéristiques essentielles que constitue la formation de complexes entre les facteurs LAF, et notamment les facteurs PE, d'une part, et les anticorps anti-MDP, de préférence les anticorps "sélectifs", d'autre part, et la récupération des facteurs LAF et, le cas échéant, PE eux-mêmes, à partir de ces complexes, après dissociation de ceux-ci.

Il est avantageux d'avoir recours à des anticorps sélectifs anti-MDP supportés par un support insoluble en milieu aqueux. A cet effet, on peut avoir recours à tout support, de préférence pulvérulent, sur lequel les anticorps anti-MDP peuvent être fixés sans que soit mise en cause leur capacité de former des complexes avec le facteur LAF isolé. On préfère avoir recours à des anticorps anti-MDP sélectifs fixés sur le matériau support commercialisé sous la désignation SEPHAROSE 4B, après activation de celui-ci au bromure de cyanogène. Bien entendu, d'autres supports peuvent être envisagés tels que billes de verre, agarose et SEPHADEX, étant naturellement entendu qu'il appartiendra au spécialiste de procéder aux essais de fixation des anticorps sélectifs anti-MDP et de vérification de la préservation de l'activité complexante des anticorps anti-MDP ainsi fixés.

Les opérations de récupération du facteur PE à partir du complexe préalablement formé entre celui-ci et des anticorps sélectifs anti-MDP peuvent être conduites de toute façon en soi connue, par exemple par acidification du milieu, notamment à un pH inférieur à 4, ou par accroissement de la concentration ionique du milieu, grâce à des sels ioniques fortement solubles, par exemple tels que le thiocyanate de sodium, le chlorure de soude et le thiocyanate d'ammonium. Ces méthodes sont particulièrement avantageuses, surtout lorsque les anticorps anti-MDP auront au préalable été fixés sur un support insoluble approprié. On peut avantageusement utiliser un anticorps anti-MDP obtenu à partir d'une solution d'anticorps anti-MDP polyclonaux, préalablement purifiée par chromatographie affine sur une colonne de SEPHAROSE 4B-MDP-lysine, les anticorps anti-MDP étant ensuite élués de la colonne, notamment dans les conditions qui ont été indiquées plus haut. D'une façon générale, on peut avoir recours, pour la fixation des anticorps anti-MDP sur support insoluble, à toute technique classique de fixation d'anticorps de ce type, par exemple telle que décrite dans "Chromatographie d'affinité : Principes et Méthodes", Pharmacia.

Le milieu contenant lesdits facteurs LAF dont ils peuvent être extraits par le procédé selon l'invention peut être constitué par le surnageant de toute culture de lignées cellulaires susceptibles de produire des LAF, IL-1 ou PE sous l'effet d'une immunostimulation telle que ci-dessus définie. Il s'agit principalement de monocytes, macrophages, susceptibles d'être obtenus notamment à partir du sang périphérique, de la rate, du poumon, du péritoine et de la moelle osseuse.

Il peut être fait appel à des cellules de toutes origines, notamment des cellules humaines, de souris, de rat, de cobaye, de singe, de lapin ou de chien, en cultures primaires ou en lignées continues.

Une source de cellules activables est aussi constituée par les cellules susmentionnées, dont ont été séparés auparavant les éléments non adhérents aux flacons de culture du type de ceux commercialisés sous la marque NUNCLON ou FALCON. On peut notamment, pour les sources de cellules activables, se référer encore à l'article de I.GERY et B.H. WAKSMAN, intitulé "Potentiation of the T-lymphocyte Response to Mitogens", publié dans "The Journal of Experimental Medicine", volume 136, 1972, p. 143-155, et à l'ouvrage édité par E. PICK avec la collaboration de M. LANDY, intitulé "Lymphokine Reports 1", Academic Press, Inc.

Les milieux contenant lesdits LAF et, le cas échéant, PE auxquels le procédé selon l'invention est applicable peuvent encore être constitués par des fractions enrichies desdits surnageants, par exemple obtenus par dialyse de ces derniers pour en éliminer les teneurs en sels minéraux et en "petites molécules", par exemple celles ayant un poids moléculaire inférieur à environ 12.000.

L'invention sera davantage encore illustrée par la description des techniques qui ont permis la détermination de la capacité de reconnaissance des facteurs LAF, PE ou analogues par des anticorps sélectifs anti-MDP.

### 1° Production d'anticorps monoclonaux

#### a) Immunisation des souris

Deux groupes de souris femelles BALB/c, âgées de deux mois, ont été immunisés avec du MDP préalablement fixé sur de la sérumalbumine bovine (MDP-BSA). Le premier groupe reçoit deux injections intradermiques décalées l'une de l'autre par un intervalle de trois semaines, de 100 µg de MDP-BSA émulsifiés avec de l'adjuvant complet de FREUND (CFA). Le second groupe subit le même traitement, par voie intrapéritonéale, et avec des doses de 100 µg de MDP-BSA dans le tampon PBS.

#### b) Fusion des cellules et culture des hybrides cellulaires (hybridomes)

Neuf semaines après la dernière immunisation, les souris reçoivent deux rappels à un jour d'intervalle, avec 100 µg de MDP-BSA, en milieu salin, cette fois-ci par voie intrapéritonéale. Trois jours après le dernier rappel, on sacrifie les souris, récupère les cellules spléniques et réalise une fusion cellulaire de ces cellules spléniques avec une lignée de cellules de myélome, par exemple du myélome NSO/1. Il va naturellement de soi que l'on peut utiliser tout autre type accessible de cellules de myélome susceptibles de former, par fusion avec des cellules spléniques, des hybrides cellulaires capables d'induire la production d'ascites chez l'animal alors susceptibles d'être utilisés comme source des anticorps présentant les caractéristiques de ceux initialement produits par les cellules spléniques mises en jeu dans la fusion.

La fusion cellulaire de 100 millions de cellules spléniques de chaque groupe de souris avec $10^7$ cellules NSO/1 en présence d'une solution de polyéthylène glycol 1 500 à 41 %, peut être réalisée selon la technique décrite par Z. ESHHAR et coll. dans J. Immunol. 124, 775, 1980. Après fusion, les cellules sont distribuées dans trois à quatre microplaques à 96 puits, puis sélectionnées dans un milieu de EAGLES, modifié par DULBECCO, contenant les constituants caractéristiques du milieu HAT, ayant une teneur en hypoxanthine, aminoptérine et thymidine (HAT-DMEM) et une forte teneur en glucose, milieu qui en outre est complété pour contenir 15 % de sérum de cheval, 50 unités par ml de pénicilline et de streptomycine, 1 mM de pyruvate de sodium et 2 nM de glutamine.

Après deux semaines de culture, les hybrides cellulaires sont transférés dans un milieu HT-DMEM. Les cellules qui se sont développées, peuvent ensuite être cultivées en routine dans un milieu à base seulement de DMEM et de sérum de cheval.

Tri des clônes fabriquant des anticorps anti-MDP sélectifs

De 10 à 15 jours après l'opération de fusion, on fait le tri des hybrides susceptibles de produire des anticorps anti-MDP, en ayant recours à la technique dite de "radioimmunoassay" en phase solide, sur microplaques de poly(chlorure de vinyle) dont les puits avaient au préalable été revêtus avec du MDP fixé sur multi(poly-D-L-alanyl)-(poly-L-lysine) : MDP-A--L, à raison de 100 µl d'une solution de 25 µg/ml de MDP-A--L par puits. Après incubation à la température ambiante, pendant une heure, les microplaques sont lavées trois fois avec une solution de sérum de cheval à 1 % dans le tampon PBS (PBS-HS). 50 µl de surnageants des cultures d'hybridomes sont alors ajoutés dans chacun des puits et incubés pendant 2 heures à la température ambiante. Après trois lavages avec PBS-HS, on ajoute 50 µl d'anticorps Ig de chèvre anti-souris, marqués à l'iode 125 ($^{125}$I) ($10^5$ coups par minute : cpm). On laisse incuber pendant une nuit à 4°C. Le niveau de radio-activité est déterminé dans un compteur de rayonnements Gamma après 4 lavages, séchages et découpages des puits.

Le tableau qui suit résume les "efficacités

12

de fusion" des deux essais de fusion mentionnés plus haut.

| Groupe | Immunisation | Nombre de cultures de cellules hybrides qui ont poussé | Hybridomes positifs |
|--------|-------------|-------------------------------------------------------|---------------------|
| 1 | MDP-BSA dans le CFA | 384/384 | 72/384 |
| 2 | MDP-BSA dans le PBS | 288/288 | 29/288 |

Ont été considérés comme hybridomes positifs ceux qui ont conduit à des détections de 5 000 à 15 000 cpm, eu égard à un bruit de fond de 500 cpm dans les essais réalisés avec des surnageants non producteurs d'anticorps anti-MDP.

On a réalisé une deuxième sélection d'anticorps monoclonaux anti-MDP dans des conditions semblables dans une deuxième série d'essais avec des microplaques dont les puits avaient été revêtus respectivement de MDP-A--L, de M-A--L et DP-A--L (c'est-à-dire de composés dans lesquels le MDP est remplacé par ses constituants de base : acide muramique (M) et L-alanyl-D-isoglutamine (DP)). Les anticorps monoclonaux actifs à l'égard de MDP-A--L, qui ne présentaient cependant que peu ou pas d'activité à l'égard de DP-A--L ou de M-A--L, ont été sélectionnés.

Pour produire des quantités d'anticoprs plus importantes, les hybridomes sélectionnés sont administrés par voie intrapéritonéale à raison de $10^7$ cellules hybrides à des souris BALB/c x DBA/2)F1. Les anticorps secrétés sont ensuite récupérés de façon connue en soi à partir des tumeurs ascitiques fluides induites par ces hybridomes chez ces souris.

On a également sélectionné, dans les conditions sus-indiquées, les anticorps monoclonaux capables de reconnaître spécifiquement la molécule porteuse, c'est-à-

13

dire la BSA. Ces anticorps monoclonaux ont été utilisés à titre de témoins.

Pour produire des quantités d'anticorps plus importantes, les hybridomes sélectionnés sont administrés par voie intrapéritonéale à raison de $10 \times 10^6$ cellules hybrides à des souris BALB/c x DBA/2)F1. Les anticorps secrétés sont ensuite récupérés de façon connue en soi à partir des tumeurs ascitiques fluides induites par ces hybridomes chez ces souris, notamment en fractionnant l'ascite sur un gel, en particulier le gel SEPHACRYL 200, et en récupérant la bande contenant l'immunoglobuline, qui donne lieu à des réactions immunologiques croisées avec l'antigène MDP (bande correspondant à un poids moléculaire d'environ 160.000).

2° <u>Mise en évidence de la capacité des anticorps anti-MDP de se combiner au facteur PE.</u>

Pour démontrer la capacité des anticorps monoclonaux anti-MDP à se combiner sélectivement aux PE, on a eu recours à l'étude de la capacité de ces préparations d'anticorps anti-MDP d'inhiber l'augmentation de l'incorporation de thymidine $^3$H (activité spécifique 1 Ci/mMol) par des thymocytes de souris C57B1/6 âgées de 6 semaines, stimulées par les PE en présence de PHA (phytohémagglutinine), employée à la dose de 1 µg/ml, dans les conditions décrites par ROSENWASSER, L., DINARELLO, C.A., dans "Cell. Immunol.", vol. 63, 1981, p. 134-142.

Les tests basés sur la détection de l'activité LAF, basée sur l'augmentation mesurée de l'incorporation de la thymidine tritiée, sont extrêmement sensibles et impliquent des doses de PE bien plus faibles que celles qui seraient requises pour la mise en évidence de l'inhibition par les anticorps anti-MDP de l'activité pyrogène elle-même *in vivo* sur le lapin.

0101363

14

Dans les expériences d'inhibition de l'activité LAF, la fraction PL purifiée susdite, diluée au 1/100 a été préincubée pendant 1 h à 37°C puis 12 h à 4°C avec les différents anticorps, c'est-à-dire :

a) soit les différents anticorps monoclonaux anti-MDP obtenus chez la souris, diluée au 1/20 ;

b) soit la fraction immunoglobulines de lapin anti-PL ou la fraction immunoglobulines normales de lapin (dilution au 1/200).

Le mélange a ensuite été ajouté à la culture de thymocytes en présence de PHA.

Comme l'indiquent les résultats présentés dans le tableau 1, seule l'incorporation de thymidine

0101363

15

$^3$H est légèrement augmentée par l'addition de PHA. L'addition de l'anticorps monoclonal anti-MDP à la PHA ne modifie pas cet effet. En présence de PL et de PHA, on observe une augmentation de la réponse proliférative (P < 0,01). L'addition de l'anticorps monoclonal au mélange de ces deux substances entraîne une inhibition de 80 % de l'augmentation ainsi observée.

Dans une série suivante d'expériences, les effets de l'anticorps monoclonal anti-MDP sur l'activité LAF de la préparation purifiée de PL ont été comparés à ceux des autres anticorps : anticorps monoclonal anti-BSA, immunoglobulines de lapin spécifiquement anti-PL et immunoglobulines normales de lapin.

Comme le montrent les résultats présentés plus loin, aucun des antisérums témoins n'a été capable de modifier de façon significative l'incorporation de thymidine $^3$H induite par l'association PHA + PL. En revanche, l'antisérum de lapin spécifiquement anti-PL utilisé à la dilution de 1/200 entraîne une inhibition de 48 % à 38 %. Dans toutes les expériences le sérum monoclonal de souris spécifiquement anti-MDP a inhibé de façon très significative l'activité LAF de la préparation de PE purifiée (53, 34, 59 et 28 %).

Afin de vérifier que l'inhibition induite par l'anticorps monoclonal anti-MDP était spécifiquement liée à une activité anti-MDP, 100 µg/ml de MDP ont été ajoutés au moment de l'incubation du PL avec les différents antisérums, avant la stimulation des thymocytes par la PHA. Dans cette expérience répétée à trois reprises, l'addition de MDP n'a pas modifié l'inhibition de l'activité LAF induite par le sérum de lapin spécifiquement anti-PL. De même, les réponses observées avec les deux autres antisérums témoins, immunoglobulines normales de lapin et monoclonal anti-BSA, n'ont pas été modifiées. En revanche, l'inhibition induite par l'anticorps monoclonal anti-MDP a été significativement réduite (41 à 100 %) (P < 0,01).

Les résultats moyens sont rassemblés dans le tableau ci-après :

### INHIBITION PAR UN ANTICORPS MONOCLONAL ANTI-MDP
### DE L'ACTIVITE LAF *IN VITRO* DU PE

| | |
|---|---|
| Témoin | $317 \pm 38$ |
| PHA (1 µg/ml | $615 \pm 49$ |
| PHA + anti-MDP (1/20) | $569 \pm 57$ |
| PHA + PL | $2079 \pm 237$ |
| PHA + PL + anti-MDP | $921 \pm 33$ |

Il découle de ce qui précède que cette réaction de complexation sélective du facteur PL peut être mise à profit pour le séparer à partir d'un milieu le contenant, en mélange avec d'autres peptides, glycopeptides et en particulier fragments de peptidoglycanes. Il est à cet égard remarquable de noter que ces derniers ne conduisent à aucune réaction immunologique avec les anticorps anti-MDP.

Le facteur PL tel qu'il est retenu dans les complexes facteur PL - anticorps anti-MDP, peut être obtenu à partir de celui-ci par dissociation dudit complexe. Mais comme on l'a indiqué plus haut, cette dissociation pourra être réalisée beaucoup plus simplement si les anticorps anti-MDP sont utilisés sous une forme supportée sur un support insoluble.

On décrit ci-après une méthodologie susceptible d'être utilisée pour procéder à une séparation de quantités plus importantes de facteur LAF et éventuellement PE à partir d'un surnageant de culture de cellules d'une lignée granulocyto-macrophagique appropriée, en mettant en oeuvre une colonne de SEPHAROSE 4B activée au bromure de cyanogène couplé selon des techniques classiques à des anticorps monoclonaux anti-MDP.

a) Immuno-absorption du facteur LAF.

Le surnagant, le cas échéant concentré au préalable, est mis en contact avec une colonne immuno-absorbante retenant des groupes anti-MDP fixés. Après un contact suffisant à permettre la fixation sélective du facteur LAF, le matériau immuno-absorbant est lavé pour éliminer les produits retenus non spécifiques. Avantageusement, on travaille sur colonne, et les rinçages sont effectués par le passage du liquide de rinçage au travers de la colonne. Les molécules retenues sur les anticorps de la colonne, c'est-à-dire le facteur LAF et éventuellement d'autres monokines ou médiateurs de poids moléculaires distincts et susceptibles de contenir la structure MDP sous une forme accessible aux anticorps, sont éluées par un tampon à bas pH, par exemple une solution à base de glycine/HCl, pH 3,2 ou par une solution à concentration saline élevée, par exemple le thiocyanate de sodium 1M.

b) Pour le cas où d'autres molécules seraient éluées en même temps que le facteur LAF, il sera souhaitable de séparer ces molécules par chromatographie sur tamis moléculaire permettant des séparations de molécules selon leurs poids moléculaires.

Parmi les molécules éventuellement présentes, on mentionne des facteurs ayant un poids moléculaire généralement moins élevé. En conséquence, il peut être souhaitable d'avoir recours à une séparation, notamment des molécules ayant un poids moléculaire inférieur à 12.000. Toute autre méthode de séparation est envisageable.

Le procédé selon l'invention pourrait par conséquent également s'appliquer à une séparation et une purification rapide des monokines à tivité LAF, dépourvues de pyrogènes-endogènes, telles qu'elles peuvent être obtenues à partir de surnageants de populations cellulaires de lignée granulocyto-macrophagique, en présence d'ester de la N-acétyl-muramyl-L-alanyl-D-glutamine, notamment dans les conditions décrites dans la demande de brevet français déjà mentionnée n° 82 00251.

Le procédé selon l'invention permet par conséquent une séparation très sélective des facteurs LAF et plus particulièrement PE et présente par conséquent

18

le grand avantage de faciliter l'étude des autres facteurs que pouvaient initialement contenir les milieux précédents, en même temps que ces pyrogènes-endogènes.

En particulier, l'invention permet aussi l'obtention de monokines ou lymphokines distinctes, essentiellement dépourvues de pyrogènes-endogènes, monokines ou lymphokines dont l'étude peut alors être considérablement facilitée.

Le procédé selon l'invention doit trouver dés applications particulièrement avantageuses dans tout procédé d'extraction de principes actifs à partir de tout milieu également susceptible de contenir des PE ou facteurs ayant des propriétés biologiques analogues et, de ce fait, susceptibles de contaminer les produits d'extraction finals.

Par exemple, il a été montré que l'interféron α peut être produit par des leucocytes humains, après stimulation par différents agents, dont les virus des oreillons (TSUNEO, K. et MINAGAWA, T., J. Gen. Virol., 54 (1981), pages 293 à 299) ou · le virus de Sendaï (MOGENSEN, K.E., Blood Transfusion and Immunohémtatology, volume 23, n° 3,(1980), pages 373 à 397). Le système d'induction de l'interféron α est semblable à celui par lequel d'autres monokines et lymphokines sont également induites. Il consiste à prélever le "buffy coat" de sang humain centrifugé et de le conserver 24 heures à 4°C ou bien à séparer les cellules mononucléaires du sang périphérique sur un gradient de Ficoll-paque. Des leucocytes débarrassés des globules rouges sont suspendus dans un milieu de culture et incubés avec les paramyxovirus (habituellement virus de Sendaï) pendant 24 heures. Les cellules et.les débris sont centrifugés et le surnageant contient alors de l'interféron α qui peut ensuite être purifié et concentré. Cependant la plupart de ces préparations d'interféron, même après purification, contiennent de nombreux médiateurs et des protéines libérées des leucocytes pendant ces opérations. Aussi les préparations d'interféron α utilisées en clinique sont capables d'induire de fortes hyperthermies (STRANDER, H., Texas Rep. Biol. Med. vol. 35 (1977), page 429). Ces auteurs ont suggéré que ces hyperthermies pouvaient être dues à des contaminations des préparations d'interféron par des pyrogènes leucocytaires qui peuvent être produits par les mêmes stimulations *in vitro*. Par conséquent, la méthode selon l'invention,pour l'élimination de ces pyrogènes, permettrait d'obtenir un interféron de grande valeur pour les essais thérapeutiques. Aussi une méthode de séparation sélective du pyrogène-endogène des cultures leucocytaires, par passage de ce surnageant sur des colonnes contenant des anticorps anti-MDP, pourrait servir à produire de l'interféron α débarrassé d'activité pyrogène.

Comme il va de soi et comme il résulte d'ailleurs déjà de ce qui précède, l'invention ne se limite nullement à ceux de ses modes d'application et de réalisation qui ont été plus spécialement envisagés ; elle en embrasse au contraire toutes les variantes, notamment celles mettant en jeu des anticorps monoclonaux obtenus à partir d'hybridomes résultant de la fusion de cellules de myélomes avec des splénocytes de souris ou autres animaux immunisés avec tout homologue de MDP, tels que par exemple des dérivés α-ester-γ-amide de l'acide N-acétyl-muramyl-L-alanyl-D-glutamique, dans la mesure où ces anticorps permettent,comme ceux qui ont été évoqués plus haut,de procéder aux séparations qui ont été décrites, de PE, PL, LAF ou de facteurs ayant des propriétés biologiques analogues.Il va encore de soi que les anticorps monoclonaux ainsi obtenus, et plus particulièrement leur utilisation pour réaliser de telles séparations constituent des équivalents de ceux qui ont été plus particulièrement décrits et, de ce fait, ne sauraient échapper à la portée de l'invention telle qu'elle a été définie dans les revendications.

D'une façon générale, les anticorps monoclonaux susceptibles d'être mis en oeuvre dans le procédé selon l'invention peuvent être constitués par tout anticorps monoclonal reconnaissant sélectivement les homologues du MDP, caractérisé par la présence d'une

0101363

20

chaîne peptidique liée au groupe acide-N-acétyl-muramique, le premier aminoacyle de la chaîne peptidique, c'est-à-dire l'aminoacyle assurant la liaison avec le groupe N-acétyle muramique, étant choisi parmi L-alanyle, L-séryle ou L-valyle. Le deuxième aminoacyle de la chaîne peptidique n'intervient guère dans la spécificité du déterminant antigénique important à prendre en considération, pour ce qui est des anticorps monoclonaux susceptibles d'être mis en oeuvre. Les anticorps monoclonaux particulièrement sélectifs seront ceux qui, parmi ceux qui viennent d'être définis, ne reconnaissent pas la structure acide N-acétyle muramique dépourvue du groupement peptidique sus-indiqué, même à des concentrations 100 fois plus importantes que la concentration minimale nécessaire à la formation du complexe entre le MDP (ou le dérivé de MDP) et l'anticorps monoclonal correspondant.

Il résulte donc bien de ce qui précède, qu'à l'inverse, les anticorps monoclonaux susceptibles d'être mis en oeuvre peuvent également être obtenus en mettant en oeuvre initialement dans la réalisation des fusions cellulaires nécessaires à la production des hybridomes producteurs d'anticorps monoclonaux des cellules spléniques provenant d'animaux qui avaient été immunisés contre des homologues de MDP dans lesquels les fonctions α et γ-carboxyle peuvent porter des substituants différents. En particulier, on pourra avoir recours à des anticorps monoclonaux obtenus en mettant en oeuvre des diesters de l'acide N-acétyl-muramyl-L-alanyl-D-glutamique ou encore les α-esters de la N-acétyj-muramyl-L-alanyl-D-glutamine, tels qu'ils ont été décrits dans la demande de brevet européen déjà mentionnée n° 79 400357.

En ce qui concerne enfin la fabrication des hybridomes, il va de soi que l'on peut avoir recours à tout type de myélome couramment utilisé à cet effet. En particulier, on aura recours à tout myélome décrit dans la littérature technique ou dans des demandes de brevets ou brevets publiés. On mentionnera, à titre d'exemple, les myélomes plus particulièrement décrits dans les demandes de brevets européens n° 0 014 519, n° 0 018 794, n° 0 018 795 et la demande de brevet français publiée n° 78 17545/2.394.607.

21

On rappellera que le procédé décrit dans la demande de brevet français n° 82 00251 pour obtenir une fraction LAF non pyrogène, à partir du milieu de culture d'une lignée cellulaire productrice, notamment de de cellules d'une lignée granulocyto-macrophagique (tumorigène ou non), comprend l'incubation de ladite lignée cellulaire en présence d'un dérivé de MDP non pyrogène, tel que le murabutide. Le facteur LAF non pyrogène libéré dans le milieu peut ensuite être récupéré à partir de celui-ci par les techniques usuelles.

22
REVENDICATIONS

1 - Procédé de séparation ou de purification d'un facteur LAF, ayant des propriétés d'activation des lymphocytes et, le cas échéant, des propriétés pyrogènes-endogènes, à partir d'un milieu biologique liquide le contenant à l'état dissous, ou d'enrichissement de ce milieu en ce facteur LAF, caractérisé par la mise en contact de ce milieu avec des anticorps anti-MDP, et la récupération soit du facteur LAF lui-même, à partir du complexe facteur LAF-anticorps anti-MDP, soit du milieu alors substantiellement dépourvu de ce facteur.

2 - Procédé selon la revendication 1, caractérisé en ce que les anticorps anti-MDP mis en oeuvre sont essentiellement constitués par des anticorps reconnaissant spécifiquement la structure N-acétyl-muramyl-L-alanyl-D-isoglutamine dans son ensemble, à l'exclusion de la structure de l'acide N-acétyl-muramique isolée ou de celle de la L-alanyl-D-isoglutamine isolée.

3 - Procédé selon la revendication 1 ou la revendication 2, caractérisé en ce que les anticorps anti-MDP sont fixés sur un support insoluble en milieu aqueux.

4 - Procédé selon l'une quelconque des revendications 1 à 3, caractérisé en ce que l'opération de récupération du facteur LAF, à partir du complexe facteur LAF anticorps anti-MDP, comprend la mise en contact de ce dernier avec un milieu tamponné acidifié à pH inférieur à 4, ou avec une solution d'un sel ionisé à concentration élevée.

5 - Procédé selon l'une quelconque des revendications 1 à 4, caractérisé en ce que les milieux biologiques susdits, initialement mis en oeuvre sont constitués par le surnageant, éventuellement concentré au préalable, de cultures de cellules d'une lignée granulo-cytomacrophagique, en présence d'un facteur immunostimulant.

6 - Procédé selon la revendication 5, caractérisé en ce que le milieu biologique susdit est essentiellement dépourvu de **protéines ayant un poids moléculaire** inférieur à une **valeur de l'ordre de 12.000.**

7 -Procédé selon l'une quelconque des revendications 1 à 3, caractérisé en ce que le susdit milieu biologique liquide contient à la fois un principe biologique à extraire et un facteur pyrogène-endogène ayant également des propriétés LAF, et en ce que l'on recueille finalement le milieu biologique sensiblement dépourvu de pyrogène-endogène, le principe biologique pouvant ensuite être extrait dudit milieu biologique, à l'état sensiblement dépourvu de contamination pyrogène-endogène.

# Office européen des brevets

## RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | JOURNAL OF IMMUNOLOGY, vol. 118, no. 5, mai 1977, pages 1631-1638, US  G. BLYDEN et al.: "Purification and properties of human lymphocyte activating factor (LAF)" * En entier * | 1 | A 61 K   39/39 |
| | ----- | | |

**DOMAINES TECHNIQUES RECHERCHES (Int. Cl. 3)**

A 61 K

Le présent rapport de recherche a été établi pour toutes les revendications

| Lieu de la recherche | Date d'achèvement de la recherche | Examinateur |
|---|---|---|
| LA HAYE | 24-10-1983 | REMPP G.L.E. |